# FASCICULE DE BREVET EUROPEEN

(11) **EP 2 882 853 B1**
(45) Date de publication et mention de la délivrance du brevet: **31.05.2017**
(21) Numéro de dépôt: 13756916.6
(22) Date de dépôt: 06.08.2013
(51) Int. Cl.: C12N 9/24, C12N 9/26

(54) **PROCÉDÉ D'EXTRACTION DE BETA-AMYLASES A PARTIR D'UNE FRACTION SOLUBLE DE PLANTE AMIDONNIÈRE ET EN PRÉSENCE D'UNE PROTÉASE**
VERFAHREN ZUR EXTRAKTION VON BETA-AMYLASEN AUS EINER LÖSLICHEN FRAKTION EINER STÄRKEPFLANZE IN GEGENWART EINER PROTEASE
METHOD FOR EXTRACTING -AMYLASES FROM A SOLUBLE FRACTION OF A STARCH PLANT AND IN THE PRESENCE OF A PROTEASE.

(30) Priorité: 07.08.2012 FR 1257680
(43) Date de publication de la demande: 17.06.2015
(73) Titulaire: Roquette Frères, 62136 Lestrem (FR)
(72) Inventeur: COURBOIS, Vincent, F-62400 Bethune (FR); DUFLOT, Pierrick, F-62136 La Couture (FR); LECOCQ, Aline, F-62400 Bethune (FR); VERRIN, Jean-Marc, F-62660 Beuvry (FR)
(74) Mandataire: Cabinet Plasseraud
(86) Numéro de dépôt international: PCT/FR2013/051899
(87) Numéro de publication internationale: WO 2014/023915

(56) Documents cités:
- EP-A2- 1 134 288
- FR-A1- 2 943 686

## Description

La présente invention est relative à un procédé amélioré d'extraction de β-amylases à partir d'une fraction soluble de plante amidonnière, ledit procédé comprenant une étape de clarification par microfiltration et une étape de concentration / purification par ultrafiltration. Ce procédé fait appel à une protéase au cours de l'étape de microfiltration, ce qui réduit très sensiblement le colmatage des membranes utilisées : on augmente ainsi le temps de production avant lavage, et donc le rendement économique du procédé correspondant. Parallèlement, la mise en oeuvre d'une telle protéase conduit à des perméats parfaitement limpides ce qui facilite l'étape d'ultrafiltration, et permet d'améliorer la transmission en β-amylase. Enfin, la protéase n'a aucun effet négatif sur l'activité β-amylasique.

Les β-amylases sont des exo-hydrolases qui libèrent des unités maltose à partir des β-extrémités non réductrices des polymères ou oligomères de glucose liés en α 1→4, la réaction s'arrêtant au premier point de branchement α 1→6 rencontré. Composants majoritaires du « pouvoir diastatique » (correspondant aux activités combinées des α-amylases, β-amylases, α-glucosidases et enzymes débranchantes) durant le maltage (germination artificielle des graines de céréales), les activités β-amylasiques isolées de ce cocktail enzymatique sont essentielles pour la production du maltose ou d'autres sucres fermentescibles générés à partir d'amidon.

L'activité saccharifiante des seules β-amylases est donc exploitée dans bon nombre d'applications : en panification, en malterie, comme additif alimentaire, voire même comme agent digestif, pour la production d'édulcorants, en pharmacie pour la production de vaccins, et enfin pour la production de maltose et de sirops enrichis en maltose (précurseur du maltitol et des sirops de maltitol).

Les procédés de fabrication de β-amylases sont nombreux. On sait ainsi que les graines d'orge, de seigle ou de blé non germées sont autant de matériels biologiques de choix pour la préparation commerciale, à grande échelle, de β -amylases. Il est par ailleurs connu de l'homme du métier que la moitié des β-amylases extractibles des graines non germées de l'orge, du blé ou du seigle peut être facilement obtenue sous la forme d'enzymes libres par extraction avec de l'eau et des solutions salines. L'autre moitié se présente en partie sous forme « liée » qui nécessite l'addition d'agents réducteurs ou des enzymes protéolytiques pour son extraction. Une autre fraction de β-amylases non directement extractible, dite « latente » a également été décrite : il faut des détergents pour l'extraire des graines de céréales. Par ailleurs, les procédés d'extraction de la β-amylase décrits dans l'état de la technique sont adaptés en fonction de l'application visée.

A cet égard, la Demanderesse a récemment mis au point et protégé dans la Demande EP 2 414 379 un procédé original de production de β-amylases, en ce sens qu'il repose sur une matière première de départ jusqu'alors peu valorisée : les « fractions solubles ». ces dernières étaient auparavant utilisées de manière exclusive comme source d'azote en fermentation et en tant qu'aliment nutritif pour le bétail une fois lesdites fractions enrichies en fibres.

Ces fractions solubles sont produites au cours de l'extraction par voie humide des composants des plantes amidonnières, comme le maïs, la pomme de terre, la patate douce, le blé, le riz, le pois, la fève, la fèverole, le manioc, le sorgho, le konjac, le seigle, le sarrasin et l'orge. Les composants dits « nobles », produits au cours de l'extraction, sont notamment les amidons, les protéines ou encore les fibres. Les « fractions solubles » désignent par opposition des constituants « non nobles » : il s'agit des résidus liquides issus de ladite extraction, quand bien même de tels résidus peuvent encore contenir sous forme de traces des substances insolubles ainsi que des particules et des colloïdes divers et variés.

Le procédé objet de la Demande EP 2 414 379 repose sur la sélection initiale de la fraction soluble à traiter, puis sur une étape de clarification de celle-ci réalisée par microfiltration. A cette occasion, il a été démontré que la β-amylase obtenue était particulièrement bien adaptée à la préparation de sirops de maltose, au même titre qu'une β-amylase produite selon des techniques antérieures mais à partir de procédés plus complexes et plus onéreux.

Poursuivant ses travaux de développement sur cette technologie, la Demanderesse a été confrontée à un nouveau problème technique: il s'avère en effet que les substances insolubles résiduelles, les particules et les colloïdes contenus dans les « fractions solubles » utilisées, se comportent comme des « poisons » à l'égard des membranes de microfiltration en obstruant celles-ci. Le rendement du procédé est alors amoindri par les arrêts rendus obligatoires par montée en pression rapide : le nettoyage s'avère donc obligatoire. En d'autres termes, le procédé objet de la Demande EP 2 414 379 nécessite d'être amélioré de manière à résoudre le problème de colmatage des membranes de microfiltration.

Travaillant dans ce sens, la Demanderesse est parvenue à démontrer que l'utilisation d'une protéase, au cours de l'étape de clarification par microfiltration, diminuait très sensiblement les problèmes de colmatage. De façon tout à fait surprenante, il s'avère que ladite protéase n'affecte en rien les propriétés de la β-amylase : comme bien connu, lorsqu'on met en oeuvre une protéase dans un milieu contenant des protéines, dont une protéine d'intérêt, ladite protéase est susceptible de dégrader la protéine d'intérêt.

On obtient selon l'invention des perméats extrêmement limpides, et on améliore la transmission de l'activité β-amylasique. On est donc parvenu à fournir à l'homme du métier un procédé de fabrication de β-amylases parfaitement adaptées à la synthèse de sirops de maltose, à travers un procédé simple à mettre en oeuvre, tout en limitant fortement le problème de colmatage des dispositifs de microfiltration.

Aussi, un premier objet de la présente invention consiste en un procédé d'extraction de β-amylases à partir d'une fraction soluble de plante amidonnière comprenant :
a) une étape de microfiltration d'une fraction soluble de plante amidonnière,
b) suivie d'une étape d'ultrafiltration
ledit procédé étant caractérisé en ce que l'étape de microfiltration est réalisée en présence d'au moins une protéase.

Les protéases (ou peptidases ou enzymes protéolytiques) sont des enzymes qui brisent les liaisons peptidiques des protéines. On parle alors de coupure protéolytique ou de protéolyse. Ce processus implique l'utilisation d'une molécule d'eau ce qui les classe parmi les hydrolases. Les fonctions biologiques des protéases sont variées : elles interviennent dans la maturation des protéines, dans la digestion des aliments, dans la coagulation sanguine, dans le remodelage des tissus au cours du développement de l'organisme et dans la cicatrisation.

Il existe 4 grandes familles de protéases en fonction de la nature du ou des acides aminés du site actif impliqué dans la catalyse :
- les sérine protéases : enzymes de digestion chez les mammifères (ex : alcalase)
- les protéases à thiol qui possède une cystéine dans leur site actif : papaine (Lypaine™ 6500 L), bromelaine cathepsines
- les aspartyl protéases : protéases acides agissant à pH acide et possédant un acide aspartique sur leur site actif (ex : la pepsine)
- les métallo protéases qui possède un cation métallique (Zn²⁺) qui intervient pour activer une molécule d'eau qui clive la chaine peptidique (Neutrase, Brewlyve™ NP900).

En amont du procédé conforme à l'invention, il convient de choisir la fraction soluble de plantes amidonnières à traiter. Cette sélection s'opère notamment dans le groupe constitué des fractions solubles du blé, de la pomme de terre, du pois, de fève, de fèverole, du riz, de l'orge, du seigle, du sarrasin, de la patate douce et préférentiellement du blé et de l'orge.

La première étape du procédé conforme à l'invention consiste à réaliser une étape de microfiltration desdites fractions solubles en présence d'au moins une protéase. La microfiltration a notamment pour but d'éliminer les substances insolubles, les colloïdes, et le matériel microbiologique en vue d'obtenir une composition limpide contenant de la β-amylase. Cette dernière composition se trouve donc être le perméat de microfiltration. Au préalable de la microfiltration la protéase est mise en contact avec la fraction soluble de plante amidonnière à traiter : l'homme du métier saura adapter le temps de contact nécessaire à l'action de l'enzyme.

La protéase mise en oeuvre dans la présente invention est choisie parmi les sérine protéases, les protéases à thiol, les aspartyl protéases et les métallo protéases, et est plus particulièrement choisie parmi les métallo protéases. De manière nominative, les protéases préférées dans la présente invention sont les produits commercialisés sous les dénominations : Sumizyme™ APL, Lypaine™ 6500 L, Neutrase™ 0,8L, Brewlyve™ NP 900, Brewers Clarex™. On préfèrera utiliser une quantité de protéase comprise entre 0,01 % et 0,1 % en volume par rapport au volume de la fraction soluble de plante amidonnière à traiter.

L'étape de microfiltration du procédé conforme à l'invention est réalisée préférentiellement par microfiltration tangentielle membranaire. Plus particulièrement, la société Demanderesse recommande de réaliser la microfiltration tangentielle avec des membranes céramiques présentant une porosité de 0,1 µm à 1 µm.

De manière facultative, l'étape de microfiltration peut être précédée d'une étape de floculation des particules insolubles contenues dans la fraction soluble de plantes amidonnières, par toute technique connue par ailleurs de l'homme du métier.

Pour cette première étape de microfiltration, la Demanderesse recommande de travailler à un pH compris entre 4 et 5 et à une température comprise entre 40 °C et 50 °C.

L'étape de microfiltration est en particulier contrôlée par la montée de la pression transmembranaire (PTM) dans le temps, à débit de perméat fixé. La turbidité du perméat est causée par des particules colloïdales qui absorbent, diffusent et/ou réfléchissent la lumière, mais surtout par les complexes protéines-polyphénols. Elle peut être déterminée au moyen d'un turbidimètre, mais peut aussi très bien s'apprécier de manière visuelle. Quant à la pression PTM, elle est déterminée classiquement au moyen de capteurs disposés à l'entrée et à la sortie du dispositif de filtration.

L'activité enzymatique de la solution contenant la β-amylase est également évaluée. La mesure de l'activité enzymatique est déterminée par l'activité diastasique. Cette dernière est exprimée en degré de pouvoir diastasique (°DP), défini comme la quantité d'enzyme contenue dans 0,1 ml d'une solution à 5 % d'un échantillon de préparation d'enzymes suffisante pour réduire 5 ml de liqueur de Fehling, quand ledit échantillon est placé dans 100 ml du substrat pendant 1 h à 20°C. Le pouvoir diastasique permettant de mesurer toutes les activités enzymatiques de type amylasique, la société Demanderesse s'est assurée par d'autres méthodes enzymatiques plus spécifiques (par exemple en utilisant le kit de dosage MEGAZYME spécifique à l'α-amylase commercialisé par CERALPHA METHOD) que la préparation de β-amylases conforme à l'invention ne renfermait pas d'autres activités contaminantes.

On détermine ensuite la transmission comme étant le rapport d'activité β-amylasique contenue dans le perméat de microfiltration sur l'activité β-amylasique contenu dans l'alimentation de la microfiltration.

La deuxième étape du procédé selon l'invention est une étape d'ultrafiltration, visant tout d'abord à concentrer le perméat de microfiltration contenant la β-amylase, tout en le débarassant d'éventuels sels résiduels contaminants, sucres et protéines. L'ultrafiltration est ainsi réalisée sur le perméat de la microfiltration de manière à obtenir un rétentat d'ultrafiltration contenant la β-amylase. Le rétentat d'ultrafiltration est alors dialysé de façon à diminuer la concentration en impuretés dans ledit rétentat.

Plus particulièrement, la société Demanderesse recommande de réaliser l'ultrafiltration à l'aide de membranes présentant un seuil de coupure de 10 000 Da à 50 000 Da, de préférence un seuil de 30 000 Da. Les fractions solubles sont ultrafiltrées sur un module équipé de membranes polysulfonées d'un seuil de coupure de 30 000 Da en cassettes à l'échelle laboratoire et membranes spiralées polysulfonées d'un seuil de coupure de 30 000 Da à l'échelle pilote. L'enzyme se concentre alors dans le rétentat au fil du temps.

Les exemples qui suivent permettent de mieux appréhender l'invention, sans toutefois en limiter la portée.

### EXEMPLES

Dans cet exemple, on réalise tout d'abord la préparation de β-amylase selon le procédé décrit dans la Demande EP 2 414 379, c'est à dire à partir d'une étape de microfiltration de fraction soluble sans mettre en oeuvre de protéase : il s'agit de l'essai témoin. On réalise ensuite la préparation de β-amylase selon le procédé objet de la présente invention, c'est à dire par microfiltration de fraction soluble en présence d'une protéase : ces essais illustrent la présente invention.

En amidonnerie de blé, on prélève une fraction soluble à l'entrée de l'évaporateur des solubles, étape classiquement mise en oeuvre pour fabriquer des produits destinés à l'alimentation du bétail, une fois concentrés. Ces produits sont commercialisés par la société Demanderesse sous le nom de CORAMI®. Ces fractions solubles présentent un pH de 4 et une activité β-amylasique de l'ordre de 30 °DP.

On réalise ici, sur un équipement à l'échelle pilote, la microfiltration de fractions solubles de blé. L'unité de microfiltration est équipée de membranes céramiques en oxyde de titane, dont le seuil de coupure est égal à 0,2 µm. le débit de perméat est fixé à 12 L / h m². Le facteur de concentration volumique est égal à 1,5. La température et le pH du perméat sont respectivement égaux à 45°C et 4,5.

On ajoute dans la fraction soluble la protéase à tester, à une concentration fixée à 0,1 % en volume par rapport au volume total de ladite composition. On laisse au préalable agir cette protéase pendant 1 heure.

Pour chacun des essais, on suit au cours de l'étape de microfiltration l'évolution de la pression PTM, comme déjà décrite auparavant : la PTM augmente dans le temps au fur et à mesure que la membrane se colmate. Pour chaque essai, on détermine également en début d'essai (Alim °DP), après 1 heure (°DP 1 heure) et en fin de procédé après environ 8 heures (°DP 8 heures), le degré de pouvoir diastasique (°DP) ; on peut ainsi calculer la transmission de l'activité β-amylasique après 1 heure (T 1 heure) et après 8 heures (T 8 heures). Enfin, on détermine au terme de chaque essai, et de manière visuelle, le caractère turbide ou limpide du perméat.

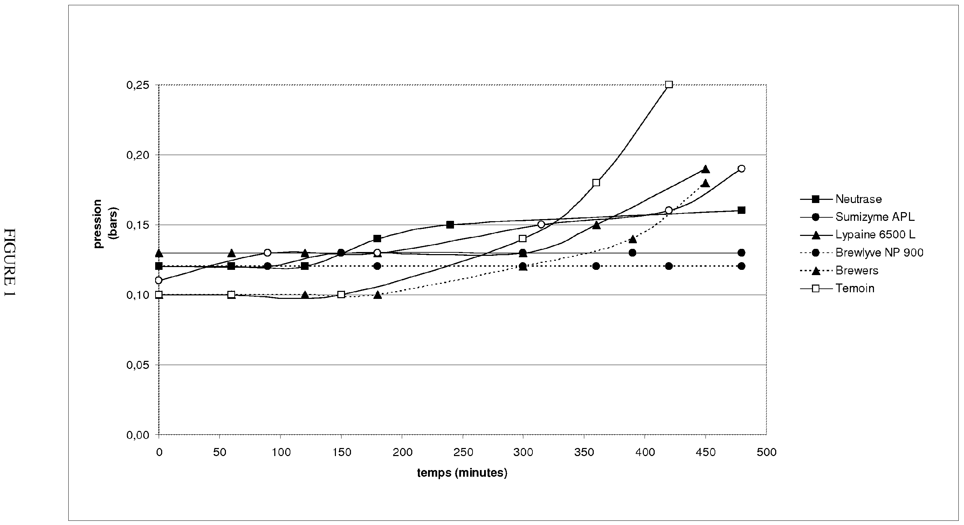

La figure 1 / 1 illustre l'évolution de la pression PTM en fonction du temps, pour l'essai témoin réalisé sans protéase, et pour chaque test représentatif de l'invention qui utilise une protéase. Il apparaît très clairement que la mise en oeuvre d'une protéase permet de limiter l'augmentation de la pression PTM dans le temps : on est donc bien parvenu à ralentir les phénomènes de colmatage de la membrane. On constate même que pour certaines protéases, dont notamment le produit Brewlyne™ NP 900, la pression transmembranaire est parfaitement stable : dans ce cas, le phénomène de colmatage de la membrane est pratiquement absent.

De plus, on note pour chaque essai selon l'invention que le perméat obtenu est parfaitement limpide. Enfin, comme le démontre le tableau 1, la transmission est améliorée dans le cas de l'invention par rapport à l'essai témoin réalisé sans protéase.

**Tableau 1**

| Protéase utilisée | Alim °DP | °DP 1 heure | T 1 heure | °DP 8 heures | T 8 heures |
|---|---|---|---|---|---|
| Aucune (témoin) | 31 | 25 | 81 | 19 | 61 |
| Sumizyne™ APL | 31 | 28 | 90 | 22 | 71 |
| Lypaine™ 6500 L | 38 | 33 | 87 | 26 | 68 |
| Brewlyne™ NP 900 | 36 | 32 | 89 | 27 | 75 |
| Brewers Clarex™ | 37 | 35 | 95 | 25 | 68 |
| Neutrase™ 0,8 L | 32 | 27 | 84 | 24 | 75 |

L'étape de microfiltration est suivie par une étape d'ultrafiltration, réalisée sur le perméat de microfiltration. Elle a pour principal objectif de concentrer ledit perméat et de le débarasser d'éventuels sels résiduels contaminants, sucres et protéines.
On récupère 40 litres de perméat de microfiltration pour chacun des essais à part l'essai témoin. Celui-ci est ultrafiltré sur un module de laboratoire MILLIPORE avec 0,18 m² de membranes présentant un seuil de coupure de 30 000 Da. On récupère 39,5 litres de perméat ultrafiltré et un rétentat concentré d'un facteur 75, présentant une activité β-amylasique comprise entre 1500 et 1600 °DP. On dialyse le rétentat d'ultrafiltration à volume constant avec 2,5 volumes d'eau en continu de façon à diminuer la concentration en impuretés des solubles d'un facteur de 10. La préparation de β-amylases ainsi obtenue est alors stockée à + 4°C.

### Exemple 2

On procède de la même manière que précédemment, mais en partant de fractions solubles présentant un pH de 4 et une activité β-amylasique de l'ordre de 30 °DP.
On ajoute dans la fraction soluble l'enzyme à tester :
- Neutrase 0,8 L (protéase) à une concentration fixée à 0,1 % en volume par rapport au volume total de ladite composition ;
- Optiflow (hemicellulase) à une concentration fixée à 1 % en volume par rapport au volume total de ladite composition ;
- Rapidase (pectinase) à une concentration fixée à 1 % en volume par rapport au volume total de ladite composition ;
- Finizyme (lysophospholipase) à une concentration fixée à 1 % en volume par rapport au volume total de ladite composition.

On laisse au préalable agir chaque enzyme pendant 1 heure.

Pour chaque essai, on détermine également en début d'essai (Alim °DP), après 1 heure (°DP 1 heure) et en fin de procédé après environ 8 heures (°DP 8 heures), le degré de pouvoir diastasique (°DP) ; on peut ainsi calculer la transmission de l'activité β-amylasique après 1 heure (T 1 heure) et après 8 heures (T 8 heures). Les résultats apparaissent dans le tableau 2.

On constate que la protéase selon l'invention permet d'améliorer de manière bien plus significative que les autres enzymes la transmission de l'activité β-amylasique. En outre, au bout de 8 heures, seul le perméat obtenu avec la protéase selon l'invention est limpide, les autres présentant un aspect trouble très marqué.

**Tableau 2**

| Enzyme utilisée | Alim °DP | °DP 1 heure | T 1 heure | °DP 8 heures | T 8 heures |
|---|---|---|---|---|---|
| Aucune (témoin) | 32 | 25 | 79 | 21 | 70 |
| Neutrase™ 0,8 L | 32 | 29 | 92 | 25 | 86 |
| Optiflow™ | 31 | 20 | 65 | 9 | 30 |
| Rapidase™ | 31 | 21 | 68 | 12 | 39 |
| Finizyme™ | 31 | 26 | 84 | 23 | 74 |

## Revendications

1. Procédé d'extraction de β-amylases à partir d'une fraction soluble de plante amidonnière comprenant :
a) une étape de microfiltration d'une fraction soluble de plante amidonnière,
b) suivie d'une étape d'ultrafiltration
ledit procédé étant **caractérisé en ce que** l'étape de microfiltration est réalisée en présence d'au moins une protéase.

2. Procédé selon la revendication 1, **caractérisé en ce que** la fraction soluble de plante amidonnière est choisie dans le groupe constitué des fractions solubles du blé, de la pomme de terre, du pois, de fève, de fèverole, du riz, de l'orge, du seigle, du sarrasin, de la patate douce et préférentiellement du blé et de l'orge.

3. Procédé selon l'une ou l'autre des revendications 1 et 2, **caractérisé en ce que** la protéase est choisie parmi les sérine protéases, les protéases à thiol, les aspartyl protéases et les métallo protéases, et plus particulièrement parmi les métallo protéases.

4. Procédé selon l'une quelconque des revendications 1 à 3, **caractérisé en ce que** l'étape de microfiltration est réalisée préférentiellement par microfiltration tangentielle membranaire.

5. Procédé selon la revendication 4, **caractérisé en ce que** la microfiltration tangentielle est réalisée avec des membranes céramiques présentant une porosité de 0,1 µm à 1 µm.

6. Procédé selon une des revendications 1 à 5, **caractérisé en ce que** l'étape de microfiltration est précédée d'une étape de floculation des particules insolubles contenues dans la fraction soluble de plantes amidonnières.

7. Procédé selon l'une quelconque des revendications 1 à 6, **caractérisé en ce que** l'étape de microfiltration est réalisée à un pH compris entre 4 et 5, et à une température comprise entre 40 °C et 50 °C.

8. Procédé selon l'une quelconque des revendications 1 à 7, **caractérisé en ce que** l'ultrafiltration est réalisée à l'aide de membranes présentant un seuil de coupure de 10 000 Da à 50 000 Da, de préférence un seuil de 30 000 Da.

9. Procédé selon la revendication 8, **caractérisé en ce que** l'ultrafiltration est réalisée à l'aide de membranes polysulfonées d'un seuil de coupure de 30 000 Da en cassettes à l'échelle laboratoire et membranes spiralées polysulfonées d'un seuil de coupure de 30 000 Da à l'échelle pilote.

## Patentansprüche

1. Verfahren zur Extraktion von Beta-Amylasen aus einem löslichen Anteil einer Stärkepflanze, umfassend:
a) einen Schritt der Mikrofiltration eines löslichen Anteils einer Stärkepflanze,
b) gefolgt von einem Schritt der Ultrafiltration,
wobei das Verfahren **dadurch gekennzeichnet ist, dass** der Schritt der Mikrofiltration in Anwesenheit wenigstens einer Protease erfolgt.

2. Verfahren nach Anspruch 1, **dadurch gekennzeichnet, dass** der lösliche Anteil einer Stärkepflanze gewählt ist aus der Gruppe, bestehend aus löslichen Anteilen von Weizen, Kartoffel, Erbse, Bohne, Ackerbohne, Reis, Gerste, Roggen, Buchweizen, Süßkartoffel und vorzugsweise Weizen und Gerste.

3. Verfahren nach einem der Ansprüche 1 und 2, **dadurch gekennzeichnet, dass** die Protease gewählt ist aus Serinproteasen, Thiol-Proteasen, Aspartyl-Proteasen und Metallproteasen, und insbesondere aus Metallproteasen.

4. Verfahren nach einem der Ansprüche 1 bis 3, **dadurch gekennzeichnet, dass** der Schritt der Mikrofiltration vorzugsweise als Tangential-Membranmikrofiltration durchgeführt wird.

5. Verfahren nach Anspruch 4, **dadurch gekennzeichnet, dass** die Tangential-Mikrofiltration mit Keramikmembranen mit einer Porosität von 0,1 µm bis 1 µm durchgeführt wird.

6. Verfahren nach einem der Ansprüche 1 bis 5, **dadurch gekennzeichnet, dass** dem Schritt der Mikrofiltration ein Schritt der Ausflockung von in dem löslichen Anteil einer Stärkepflanze enthaltenen unlöslichen Partikeln vorangeht.

7. Verfahren nach einem der Ansprüche 1 bis 6, **dadurch gekennzeichnet, dass** der Schritt der Mikrofiltration bei einem pH-Wert von 4 bis 5 bei einer Temperatur von 40 °C bis 50 °C durchgeführt wird.

8. Verfahren nach einem der Ansprüche 1 bis 7, **dadurch gekennzeichnet, dass** die Ultrafiltration mittels Membranen durchgeführt wird, die eine Schnittschwelle von 10 000 Da bis 50 000 Da, vorzugsweise einen Schwellenwert von 30 000 Da aufweist.

9. Verfahren nach Anspruch 8, **dadurch gekennzeichnet, dass** die Ultrafiltration mittels polysulfonierter Membranen mit einer Schnittschwelle von 30 000 Da in Kassetten im Labormaßstab und polysulfonierter Spiralmembranen mit einer Schnittschwelle von 30 000 Da im Pilotmaßstab durchgeführt wird.

## Claims

1. A method for extracting β-amylases from a soluble fraction of a starch plant, comprising:
a) a step of microfiltration of a soluble fraction of a starch plant,
b) followed by an ultrafiltration step,
said method being **characterized in that** the microfiltration step is carried out in the presence of at least one protease.

2. The method as claimed in claim 1, **characterized in that** the soluble fraction of a starch plant is chosen from the group consisting of the soluble fractions of wheat, of potato, of pea, of broad bean, of horse bean, of rice, of barley, of rye, of buckwheat and of sweet potato, and preferentially of wheat and of barley.

3. The method as claimed in either of claims 1 and 2, **characterized in that** the protease is chosen from serine proteases, thiol proteases, aspartyl proteases and metalloproteases, and more particularly from metalloproteases.

4. The method as claimed in any one of claims 1 to 3, **characterized in that** the microfiltration step is preferentially carried out by tangential membrane microfiltration.

5. The method as claimed in claim 4, **characterized in that** the tangential microfiltration is carried out with ceramic membranes having a porosity of 0.1 µm to 1 µm.

6. The method as claimed in one of claims 1 to 5, **characterized in that** the microfiltration step is preceded by a step of flocculation of the insoluble particles contained in the soluble fraction of starch plants.

7. The method as claimed in any one of claims 1 to 6, **characterized in that** the microfiltration step is carried out at a pH of between 4 and 5 and at a temperature of between 40°C and 50°C.

8. The method as claimed in any one of claims 1 to 7, **characterized in that** the ultrafiltration is carried out using membranes which have a cut-off threshold of 10 000 Da to 50 000 Da, preferably a threshold of 30 000 Da.

9. The method as claimed in claim 8, **characterized in that** the ultrafiltration is carried out using polysulfone membranes having a cut-off threshold of 30 000 Da in cassettes on a laboratory scale and polysulfone spiral membranes having a cut-off threshold of 30 000 Da on a pilot scale.
